# EUROPÄISCHE PATENTANMELDUNG

(11) **EP 4 725 934 A1**
(43) Veröffentlichungstag der Anmeldung: **15.04.2026**
(21) Anmeldenummer: 24206052.3
(22) Anmeldetag: 11.10.2024
(51) Int. Cl.: C07C 45/50, C07D 317/26, C07C 47/02, C07C 47/277, C07C 47/228, C07C 47/225

(54) **VERFAHREN ZUR HYDROFORMYLIERUNG VON OLEFINEN UNTER EINSATZ VON 1,8-DIAZABICYCLO[5.4.0]UNDEC-7-EN (DBU)**

(71) Anmelder: Evonik Oxeno GmbH & Co. KG, 45772 Marl (DE)
(72) Erfinder: FRANKE, Professor, Dr., Robert, 45772 Marl (DE); JACKSTELL, Dr., Ralf, 18106 Rostock (DE); BELLER, Professor Matthias, 18211 Ostseebad Nienhagen (DE); VELLALA SYED ALI, Mohamed Niyaz, 639205 Karur, Tamil Nadu (IN)
(74) Vertreter: Evonik Patent Association

(57) **Zusammenfassung**

Verfahren zur Hydroformylierung von Olefinen unter Einsatz von 1,8-Diazabicyclo[5.4.0]undec-7-en (DBU).

## Beschreibung

Die vorliegende Erfindung betrifft ein Verfahren zur Hydroformylierung von Olefinen unter Einsatz von 1,8-Diazabicyclo[5.4.0]undec-7-en (DBU).

Die technische Aufgabe der Erfindung ist die Bereitstellung eines Verfahrens, mit welchem bei der Hydroformylierung von Olefinen eine gute Ausbeute erzielt werden kann.

Die Aufgabe wird gelöst durch ein Verfahren nach Anspruch 1.

Verfahren umfassend die Verfahrensschritte:
a) Vorlegen eines Olefins;
b) Zugabe einer Ru-Verbindung;
c) Zugabe von 1,8-Diazabicyclo[5.4.0]undec-7-en (DBU);
d) Zuführen von CO und H₂;
e) Erwärmen des Reaktionsgemisches aus a) bis d), wobei das Olefin zu einem Aldehyd umgesetzt wird.

In einer Variante des Verfahrens ist das Olefin ausgewählt aus den Substanzen von **(A)** bis **(H):**

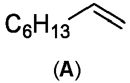

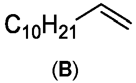

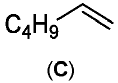

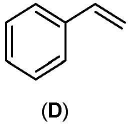

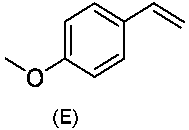

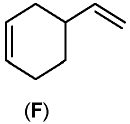

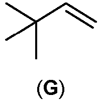

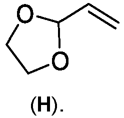

In einer Variante des Verfahrens ist die Ru-Verbindung ausgewählt aus: Ru₃(CO)₁₂, RuCl₃ x 3H₂O, [Ru(Cymen)Cl₂]₂, RuBr₃ x 3H₂O, RuI₃, Ru(PPh₃)₃Cl₂.

In einer Variante des Verfahrens handelt es sich bei der Ru-Verbindung um Ru₃(CO)₁₂.

In einer Variante des Verfahrens wird das 1,8-Diazabicyclo[5.4.0]undec-7-en (DBU) in einer Menge zugegeben, welche im Bereich von 0,1 mol% bis 10 mol%, bezogen auf das Olefin, liegt.

In einer Variante des Verfahrens werden CO und H₂ in einem mol-Verhältnis zugeführt, welches im Bereich von 1:1 bis 1:10 liegt.

In einer Variante des Verfahrens werden CO und H₂ in einem mol-Verhältnis zugeführt, welches im Bereich von 1:2 bis 1:8 liegt.

In einer Variante des Verfahrens werden CO und H₂ mit einem Druck zugeführt, welcher im Bereich von 1 MPa (10 bar) bis 10 MPa (100 bar) liegt.

In einer Variante des Verfahrens werden CO und H₂ mit einem Druck zugeführt, welcher im Bereich von 2 MPa (20 bar) bis 6 MPa (60 bar) liegt.

In einer Variante des Verfahrens wird das Reaktionsgemisch auf eine Temperatur erwärmt, welche im Bereich von 60 °C bis 180 °C liegt.

In einer Variante des Verfahrens wird das Reaktionsgemisch auf eine Temperatur erwärmt, welche im Bereich von 80 °C bis 160 °C liegt.

In einer Variante des Verfahrens weist das Verfahren den zusätzlichen Verfahrensschritt d') auf:
d') Zugabe eines Lösungsmittels.

In einer Variante des Verfahrens ist das Lösungsmittel ausgewählt aus: N-Methyl-2-pyrrolidon (NMP), HzO.

In einer Variante des Verfahrens ist das Lösungsmittel N-Methyl-2-pyrrolidon (NMP).

In einer Variante des Verfahrens weist das Reaktionsgemisch, außer den in den zuvor beschriebenen Verfahrensschritten zugeführten Verbindungen, keine weiteren Verbindungen auf, welche in einem Ru-Ligand-Komplex als Liganden an das Ru-Metall koordinieren könnten.

In einer Variante des Verfahrens ist das Reaktionsgemisch frei von Phosphororganischen Verbindungen.

In einer Variante des Verfahrens ist das Reaktionsgemisch frei von Organo-Phosphin-Verbindungen und Organo-Phosphit-Verbindungen.

In einer Variante des Verfahrens ist das Reaktionsgemisch frei von Verbindungen, welche eine P-C-Bindung oder eine P-C-O-Bindung aufweisen.

In einer Variante des Verfahrens ist das Reaktionsgemisch frei von P-haltigen Verbindungen.

Im Folgenden soll die Erfindung anhand von Ausführungsbeispielen näher erläutert werden.

### Variation der Base

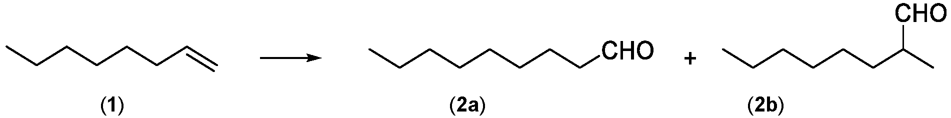

### Versuchsbeschreibung

### Allgemein

Die festen Ausgangsmaterialien Ruthenium-Vorläufer und Base werden gewogen und in ein 4,0-ml-Schraubdeckel-Vial mit einem 6-mm-Magnetrührer in eine Glovebox gegeben. Das Vial wird mit einem Teflon/Silikon-Septum und einer Phenol-Kunststoffkappe verschlossen, bevor es aus der Glovebox entnommen wird. Das Vial wird dann unter Argonzufuhr mit einer Nadel an die Schlenk-Leitung angeschlossen. Die gewünschte Menge an destilliertem Olefin und Lösungsmittel wird mit einer Mikroliterspritze injiziert. Das Fläschchen wird auf eine Legierungsplatte gesetzt und in einen Autoklaven der Serie Parr 4560 (300,0 ml) gegeben. Bei Raumtemperatur wird der Autoklav dreimal mit Stickstoffgas gespült. Anschließend wird auch Kohlenmonoxid zweimal gespült und auf einen bestimmten Druck gebracht. Dann wird Wasserstoff bis zu einem bestimmten Gesamtdruck hinzugefügt. Die Reaktion wird auf eine bestimmte Temperatur erhitzt und für eine bestimmte Reaktionszeit gerührt (450 U/min). Der Autoklav wird dann mit Eiswasser gekühlt und das Gas vorsichtig abgelassen. Isooktan (33,0 µl, 23,0 mg, 0,2 mmol) wird der Lösung als interner Standard hinzugefügt, die Lösung wird durch Celite filtriert und gaschromatographisch analysiert.

### 1,8-Diazabicyclo[5.4.0]undec-7-en (DBU)

Die festen Ausgangsmaterialien Ruthenium-Vorläufer Ru₃(CO)₁₂ (1,9 mg, 0,003 mmol) und Base 1,8-Diazabicyclo[5.4.0]undec-7-en (DBU) (6,0 mg, 0,04 mmol) werden abgewogen und in ein 4,0-mL-Schraubdeckel-Vial mit einem 6-mm-Magnetrührer in eine Glovebox gegeben. Das Fläschchen wird mit einem Teflon/Silikon-Septum und einer Phenol-Kunststoffkappe verschlossen, bevor es aus der Glovebox entnommen wird. Das Fläschchen wird dann unter Argonzufuhr mit einer Nadel an die Schlenk-Leitung angeschlossen. Die gewünschte Menge an destilliertem 1-Octen (157,0 µl, 1,0 mmol) und einer Mischung aus NMP (N-Methyl-2-pyrrolidon) und H₂O (2,0 ml, 40:1) wird mithilfe einer Mikroliterspritze injiziert. Das Fläschchen wird auf eine Legierungsplatte gesetzt und in einen Autoklaven der Serie Parr 4560 (300,0 ml) gegeben. Bei Raumtemperatur wird der Autoklav dreimal mit Stickstoffgas gespült. Anschließend wird Kohlenmonoxid ebenfalls zweimal gespült und auf 10 bar Druck gebracht. Dann wird Wasserstoff bis zu einem Gesamtdruck von 40 bar hinzugefügt. Die Reaktion wird auf 120 °C erhitzt und 24 Stunden lang gerührt (450 U/min). Der Autoklav wird dann mit Eiswasser gekühlt und das Gas vorsichtig abgelassen. Isooktan (33,0 µL, 23,0 mg, 0,2 mmol) wird der Lösung als interner Standard hinzugefügt, die Lösung wird durch Celite filtriert und gaschromatographisch analysiert.

Die Versuche mit den Vergleichsbasen wurden analog durchgeführt.

Die Ergebnisse sind in Tabelle 1 zusammengestellt:

**Tabelle 1:**

| Base | Ausbeute **(2a** + **2b)** [%] |
|---|---|
| 1,8-Diazabicyclo[5.4.0]undec-7-en (DBU)* | 53 |
| 2,6-Di-tert-butylpyridin | 31 |
| 1,10-Phenanthrolin | 41 |
| N,N,N',N'-Tetramethylethane-1,2-diamin (TMEDA) | 44 |
| N,N-Dimethylpyridin-4-amin (DMAP) | 46 |
| N,N-Diethylethanamin (TEA) | 46 |
| 1,5-Diazabicyclo[4.3.0]non-5-en (DBN) | 49 |
| Lithium N-(propan-2-yl)propan-2-aminid (LDA) | 42 |

| | |
|---|---|
| * erfindungsgemäßes Verfahren | |

### Variation des Olefins

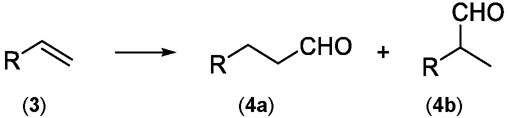

### Versuchsbeschreibung

Die festen Ausgangsmaterialien Ruthenium-Vorläufer Ru₃(CO)₁₂ (1,9 mg, 0,003 mmol) und Base 1,8-Diazabicyclo[5.4.0]undec-7-en (DBU) (3,0 mg, 0,02 mmol) werden abgewogen und in ein 4,0-mL-Schraubdeckel-Vial mit einem 6-mm-Magnetrührer in eine Glovebox gegeben. Das Fläschchen wird mit einem Teflon/Silikon-Septum und einer Phenol-Kunststoffkappe verschlossen, bevor es aus der Glovebox entnommen wird. Das Fläschchen wird dann unter Argonzufuhr mit einer Nadel an die Schlenk-Leitung angeschlossen. Die gewünschte Menge an destilliertem 1-Octen (157,0 µl, 1,0 mmol) und NMP (N-Methyl-2-pyrrolidon) (2,0 ml) wird mithilfe einer Mikroliterspritze injiziert. Das Fläschchen wird auf eine Legierungsplatte gesetzt und in einen Autoklaven der Serie Parr 4560 (300,0 ml) gegeben. Bei Raumtemperatur wird der Autoklav dreimal mit Stickstoffgas gespült. Anschließend wird Kohlenmonoxid zweimal gespült und auf 5 bar Druck gebracht. Dann wird Wasserstoff bis zu einem Gesamtdruck von 40 bar hinzugefügt. Die Reaktion wird auf 120 °C erhitzt und 24 Stunden lang gerührt (450 U/min). Der Autoklav wird dann mit Eiswasser abgekühlt und das Gas vorsichtig abgelassen. Isooktan (33,0 µL, 23,0 mg, 0,2 mmol) wird der Lösung als interner Standard hinzugefügt, die Lösung wird durch Celite filtriert und gaschromatographisch analysiert.

Die Versuche mit den weiteren Olefinen wurden analog durchgeführt.

### Reaktionsbedingungen:

Ru₃(CO)₁₂ (0,3 mol%), DBU (2,0 mol%), NMP (2,0 mL), CO/H₂ (40 bar, 1:7), 120 °C, 24 h

Die Ergebnisse sind in Tabelle 2 zusammengestellt:

**Tabelle 2:**

| Olefin | Ausbeute **(4a** + **4b)** [%] |
|---|---|
| 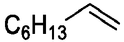 | 68 |
| 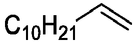 | 56 |
| 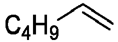 | 72 |
| 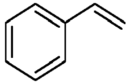 | 82 |
| 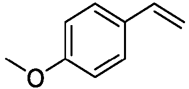 | 40 |
| 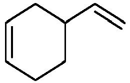 | 73 |
| 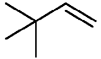 | 44 |
| 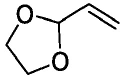 | 97 |

Wie die durchgeführten Versuche zeigen, wird die Aufgabe durch ein erfindungsgemäßes Verfahren gelöst.

## Patentansprüche

1. Verfahren umfassend die Verfahrensschritte:
a) Vorlegen eines Olefins;
b) Zugabe einer Ru-Verbindung;
c) Zugabe von 1,8-Diazabicyclo[5.4.0]undec-7-en (DBU);
d) Zuführen von CO und H₂;
e) Erwärmen des Reaktionsgemisches aus a) bis d), wobei das Olefin zu einem Aldehyd umgesetzt wird.

2. Verfahren nach Anspruch 1,
wobei das Olefin ausgewählt ist aus den Substanzen von **(A)** bis **(H):**

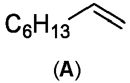

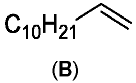

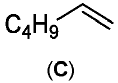

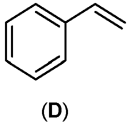

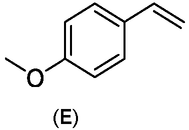

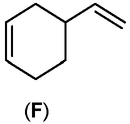

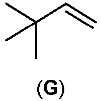

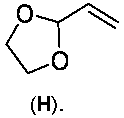

3. Verfahren nach einem der Ansprüche 1 oder 2,
wobei die Ru-Verbindung ausgewählt ist aus: Ru₃(CO)₁₂, RuCl₃ x 3H₂O, [Ru(Cymen)Cl₂]₂, RuBr₃ x 3H₂O, RuI₃, Ru(PPh₃)₃Cl₂.

4. Verfahren nach einem der Ansprüche 1 bis 3,
wobei es sich bei der Ru-Verbindung um Ru₃(CO)₁₂ handelt.

5. Verfahren nach einem der Ansprüche 1 bis 4,
wobei das 1,8-Diazabicyclo[5.4.0]undec-7-en (DBU) in einer Menge zugegeben wird, welche im Bereich von 0,1 mol% bis 10 mol%, bezogen auf das Olefin, liegt.

6. Verfahren nach einem der Ansprüche 1 bis 5,
wobei CO und H₂ in einem mol-Verhältnis zugeführt werden, welches im Bereich von 1:1 bis 1:10 liegt.

7. Verfahren nach einem der Ansprüche 1 bis 6,
wobei CO und H₂ in einem mol-Verhältnis zugeführt werden, welches im Bereich von 1:2 bis 1:8 liegt.

8. Verfahren nach einem der Ansprüche 1 bis 7,
wobei CO und H₂ mit einem Druck zugeführt werden, welcher im Bereich von 1 MPa (10 bar) bis 10 MPa (100 bar) liegt.

9. Verfahren nach einem der Ansprüche 1 bis 8,
wobei das Reaktionsgemisch auf eine Temperatur erwärmt wird, welche im Bereich von 60 °C bis 180 °C liegt.

10. Verfahren nach einem der Ansprüche 1 bis 9,
wobei das Verfahren den zusätzlichen Verfahrensschritt d') aufweist:
d') Zugabe eines Lösungsmittels.

11. Verfahren nach Anspruch 10,
wobei das Lösungsmittel ausgewählt ist aus: N-Methyl-2-pyrrolidon (NMP), H₂O.

12. Verfahren nach einem der Ansprüche 10 oder 11,
wobei das Lösungsmittel N-Methyl-2-pyrrolidon (NMP) ist.

13. Verfahren nach einem der Ansprüche 1 bis 12,
wobei das Reaktionsgemisch, außer den in den zuvor beschriebenen Verfahrensschritten zugeführten Verbindungen, keine weiteren Verbindungen aufweist, welche in einem Ru-Ligand-Komplex als Liganden an das Ru-Metall koordinieren könnten.

14. Verfahren nach einem der Ansprüche 1 bis 13,
wobei das Reaktionsgemisch frei von Phosphororganischen Verbindungen ist.

15. Verfahren nach einem der Ansprüche 1 bis 14,
wobei das Reaktionsgemisch frei von Organo-Phosphin-Verbindungen und Organo-Phosphit-Verbindungen ist.

## Geänderte Patentansprüche

### Geänderte Patentansprüche gemäss Regel 137(2) EPÜ.

1. Verfahren umfassend die Verfahrensschritte:
a) Vorlegen eines Olefins;
b) Zugabe einer Ru-Verbindung;
c) Zugabe von 1,8-Diazabicyclo[5.4.0]undec-7-en (DBU);
d) Zuführen von CO und H₂;
e) Erwärmen des Reaktionsgemisches aus a) bis d), wobei das Olefin zu einem Aldehyd umgesetzt wird;
wobei das Reaktionsgemisch frei von Phosphororganischen Verbindungen ist.

2. Verfahren nach Anspruch 1,
wobei das Olefin ausgewählt ist aus den Substanzen von (A) bis (H):

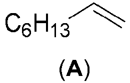

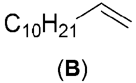

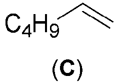

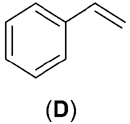

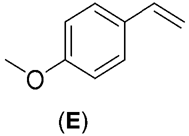

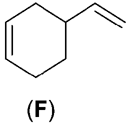

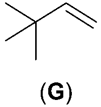

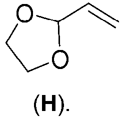

3. Verfahren nach einem der Ansprüche 1 oder 2,
wobei die Ru-Verbindung ausgewählt ist aus: Ru₃(CO)₁₂, RuCl₃ x 3H₂O, [Ru(Cymen)Cl₂]₂, RuBr₃ x 3H₂O, RuI₃, Ru(PPh₃)₃Cl₂.

4. Verfahren nach einem der Ansprüche 1 bis 3,
wobei es sich bei der Ru-Verbindung um Ru₃(CO)₁₂ handelt.

5. Verfahren nach einem der Ansprüche 1 bis 4,
wobei das 1,8-Diazabicyclo[5.4.0]undec-7-en (DBU) in einer Menge zugegeben wird, welche im Bereich von 0,1 mol% bis 10 mol%, bezogen auf das Olefin, liegt.

6. Verfahren nach einem der Ansprüche 1 bis 5,
wobei CO und H₂ in einem mol-Verhältnis zugeführt werden, welches im Bereich von 1:1 bis 1:10 liegt.

7. Verfahren nach einem der Ansprüche 1 bis 6,
wobei CO und H₂ in einem mol-Verhältnis zugeführt werden, welches im Bereich von 1:2 bis 1:8 liegt.

8. Verfahren nach einem der Ansprüche 1 bis 7,
wobei CO und H₂ mit einem Druck zugeführt werden, welcher im Bereich von 1 MPa (10 bar) bis 10 MPa (100 bar) liegt.

9. Verfahren nach einem der Ansprüche 1 bis 8,
wobei das Reaktionsgemisch auf eine Temperatur erwärmt wird, welche im Bereich von 60 °C bis 180 °C liegt.

10. Verfahren nach einem der Ansprüche 1 bis 9,
wobei das Verfahren den zusätzlichen Verfahrensschritt d') aufweist: d') Zugabe eines Lösungsmittels.

11. Verfahren nach Anspruch 10,
wobei das Lösungsmittel ausgewählt ist aus: N-Methyl-2-pyrrolidon (NMP), H₂O.

12. Verfahren nach einem der Ansprüche 10 oder 11,
wobei das Lösungsmittel N-Methyl-2-pyrrolidon (NMP) ist.

13. Verfahren nach einem der Ansprüche 1 bis 12,
wobei das Reaktionsgemisch frei von Organo-Phosphin-Verbindungen und Organo-Phosphit-Verbindungen ist.
